# EUROPÄISCHE PATENTSCHRIFT

(11) **EP 2 254 634 B1**
(45) Veröffentlichungstag und Bekanntmachung des Hinweises auf die Patenterteilung: **22.05.2013**
(21) Anmeldenummer: 09719496.3
(22) Anmeldetag: 11.03.2009
(51) Int. Cl.: A61M 15/00

(54) **INHALATOR UND SIEB FÜR EINEN INHALATOR**
INHALER AND SIEVE FOR AN INHALER
INHALATEUR ET FILTRE POUR UN INHALATEUR

(30) Priorität: 13.03.2008 DE 102008014025
(43) Veröffentlichungstag der Anmeldung: 01.12.2010
(60) Teilanmeldung: 11194615.8
(73) Patentinhaber: Boehringer Ingelheim Pharma GmbH & Co. KG, 55216 Ingelheim am Rhein (DE)
(72) Erfinder: HAERDER, Lukas, 97616 Bad Neustadt / Saale (DE); BREUER, Claus, 59320 Ennigerloh (DE)
(74) Vertreter: Simon, Elke Anna Maria
(86) Internationale Anmeldenummer: PCT/EP2009/052858
(87) Internationale Veröffentlichungsnummer: WO 2009/112521

(56) Entgegenhaltungen:
- WO-A-2004/047796
- DE-A1-102006 016 904
- DE-U1- 8 801 555
- US-A- 3 483 980
- US-A- 4 918 017
- US-A1- 2002 158 150

## Beschreibung

Die Erfindung betrifft zunächst einen Inhalator für pulverförmige, insbesondere medizinische Substanzen, mit einem zu einem Mundstück führenden Saugluftkanal, weiter einem in einer Aufnahmekammer bevorzugt beweglich vorgesehenen Substanz-Vorratsbehältnis und einem in dem Saugfuftkanal zwischen der Aufnahmekammer und dem Mundstück angeordneten Siebteil, das einen Einspannrand aufweist und einem in einem Querschnitt innerhalb des Einspannrandes verlaufenden Siebbereich, wobei der Siebbereich einen zu einer Seite hin ausladend geformten Vorstandsbereich aufweist.

Derartige Inhalatoren sind bekannt. Es wird bspw. auf die WO 2004/062716 A1 und die DE 10 2006 016904 A1 verwiesen. Das Siebteil begrenzt einseitig, Richtung Mundstück, die Aufnahmekammer. Hiermit ist es aber auch im Hinblick auf die gewünschten sehr genauen Abmessungen der Aufnahmekammer, was wiederum mit der gewünschten Beweglichkeit des Substanz-Vorratsbehältnisses zusammenhängt, Anforderungen an eine hochpräzise Maßhaltigkeit unterworfen. Bei einem dem internen Stand der Technik der Anmelderin entsprechenden Siebteil ist eine kuppelförmige Auswölbung als Vorstandsbereich vorgesehen. Die Auswölbung ist zu dem Substanz-Vorratsbehältnis hin ausgebildet. Es hat sich jedoch herausgestellt, dass fertigungstechnisch die Einleitung der erforderten Maßtoleranzen sehr schwierig ist.

Ausgehend von dem dargestellten Stand der Technik beschäftigt sich die Erfindung mit der Aufgabe, ein Sieb für einen Inhalator und einen Inhalator mit Siebteil anzugeben, wobei das Siebteil vorteilhaft gestaltet ist.

Eine mögliche Lösung der Aufgabe ist nach einem ersten Erfindungsgedanken durch den Gegenstand des Ansprucher 1 gegeben, wobei in diesem Fall darauf abgestellt ist, dass der Vorstandsbereich eine Abflachung aufweist. Überraschend hat sich herausgestellt, dass im Sinne einer erhöhten Maßhaltigkeit zwar weiter eine Ausbildung eines Vorstandsbereiches im Siebbereich des Siebteils möglich ist; dass die Maßhaltigkeit aber schon dadurch entscheidend verbessert werden kann, dass dieser Vorstandsbereich eine Abflachung aufweist. Der Vorstandsbereich ist also nicht kontinuierlich kuppelförmig gestaltet. Der Vorstandsbereich wächst vielmehr zunächst aus dem umgebenden Siebbereich heraus, knickt dann aber gleichsam, in einer Querschnittsdarstellung, im Sinne einer Abflachung ab.

Weitere Merkmale der Erfindung sind nachstehend, auch in der Figurenbeschreibung, oftmals in ihrer bevorzugten Zuordnung zu dem vorstehend behandelten Anspruchskonzept erläutert. Sie können aber auch in einer Zuordnung zu nur einer oder mehreren einzelnen Merkmalen dieses Anspruches oder unabhängig bzw. in einem anderen Gesamtkonzept von Bedeutung sein.

So ist es zunächst bevorzugt, dass der Vorstandbereich in der Querschnittsbetrachtung quer zu einer durch den Einspannrand verlaufenden Ebene über den Einspannrand hinausragt.

Der Einspannrand als solcher kann eine Abwinklung aufweisen. Die Abwinklung kann in Richtung des Vorstandsbereiches vorgesehen sein, aber auch entgegengesetzt hierzu. Die Abwinklung ist durch einen äußersten Randabschnitt des Siebteils gebildet. Das Siebteil kann insgesamt aus einem Drahtgewebe-Flachstück durch Umbiegen bzw. Tiefziehen, unter zumindest teilweise plastischer Verformung, hergestellt sein.

Weiter ist bevorzugt, dass die Abflachung in der Querschnittsbetrachtung mittig des Siebbereiches ist. Diese mittige Anordnung bezieht sich insbesondere auf ein Siebteil, das insgesamt einen kreisförmigen Grundriss aufweist. Es kann aber auch bei einem eckigen Grundriss umgebend zu dem hierbei gegebenen Mittelpunkt vorgesehen sein.

Die Abflachung als Solche hat eine Erstreckung bezogen auf eine Querschnittsdarstellung, die einem Teil des gesamten freien Abstandes zwischen gegenüberliegenden Bereichen des Einspannrandes entspricht. Bei einem kreisförmigen Durchmesser also einem Teil eines insoweit gegebenen Durchmessermaßes. Dieser Teilbereich entspricht bevorzugt 5% oder mehr des freigespannten Siebes innerhalb des Einspannrandes. Hierbei im Einzelnen bezogen auf eine Projektion einer Linie, die die gegenüberliegenden Bereiche des Einspannrandes ummittelbar miteinander verbindet. Im Falle eines rechteckigen Grundrisses ist diese Maßangabe zunächst auf die kleinste Abmessung zwischen gegenüberliegenden Bereichen des Einspannrandes bezogen. Weiter bevorzugt beträgt das Maß weniger als 15% der Gesamtabmessung der genannten Querschnittslinie. Hierbei sind, soweit eine Einschränkung auf den durch die obere und untere Schranke nunmehr genannten Bereich erfolgt, bezüglich dieses Bereiches auch alle Zwischenwerte in die Offenbarung eingeschlossen, und zwar insbesondere in 1/10%-Schritten. Die genannten Abmessungen beziehen sich auf eine Gesamtabmessung des Siebbereiches in dem angesprochenen freigespannten Bereich zwischen 5 und 15 mm. Auch diesbezüglich sind alle Zwischenwerte, insbesondere in 1/10 mm-Schritten, in die Offenbarung eingeschlossen.

Das Sieb selbst besteht bevorzugt aus metallischen Drähten. Als Werkstoff kommt insbesondere ein Edelstahlwerkstoff in Betracht, vorzugsweise mit Legierungsbestandteilen Chrom und/oder Nickel, wobei weiter bevorzugt der Chromanteil doppelt so hoch oder höher als der Nickelanteil ist.

Das Sieb besteht im Einzelnen geeigneterweise aus einem Gewebe aus den genannten Drähten. Es kann eine Maschenweite von 0,4 oder mehr Millimetern aufweisen. Weiter bevorzugt eine Maschenweite von 1,5 mm oder weniger. Darüber hinaus bevorzugt eine Maschenweite im Bereich von 0,9 -1 mm. In dem genannten Bereich von 0,m4 -1,5 mm sind hiermit auch sämtliche Zwischenwerte, und zwar insbesondere in 1/10mm-Schritten von der unteren und/oder oberen Grenze auf die jeweils andere Grenze hin einbezogen. "Und" steht hierbei dafür, dass beide Grenzen um jeweils ein bzw. mehrere Zehntel auf die jeweils andere Grenze hin verschoben, d.h. eingegrenzt werden.

Der Draht selbst kann bevorzugt einen Durchmesser zwischen 0,1 und 0,5 mm aufweisen, wobei auch hier jegliche Zwischenwerte, insbesondere in 1/10mm-Schritten, in die Offenbarung mit einbezogen sind.

Im Weiteren betrifft die Erfindung ein Siebteil für einen Inhalator, insbesondere einen Inhala tor in einer der Ausgestaltungen, wie er vorstehend beschrieben worden ist, wobei das Siebteil einen Einspannrand und einen in einem Querschnitt innerhalb des Einspannrandes verlaufenden Siebbereich aufweist, wobei weiter der Siebbereich einen zu einer Seite hin ausladend geformten Vorstandsbereich aufweist.

Bezüglich des Siebteils stellt sich die Aufgabe, dieses im Hinblick auf einen Einsatz in einem Inhalator, insbesondere einem Pulverinhalator, günstig zu gestalten.

Eine mögliche Lösung dieser Aufgabe ist beim Gegenstand des Anspruches 11 gegeben, wobei in diesem Fall darauf abgestellt ist, dass der Vorstandsbereich eine Abflachung aufweist. Zu den hiermit insbesondere im Zusammenhang mit einem Pulverinhalator erzielbaren Vorteilen wird auch auf die Ausführungen eingangs bezüglich des Inhalators insgesamt verwiesen. Desgleichen auch bezüglich der üblichen Ausgestaltungen des Siebteils.

Nachstehend ist die Erfindung des Weiteren anhand der beigefügten Zeichnung, die jedoch lediglich ein Ausführungsbeispiel darstellt, erläutert. Hierbei zeigt:
- Fig. 1: einen Inhalator mit im Saugluftkanal angeordnetem Sieb;
- Fig. 2: eine Herausvergrößerung des Bereichs II in Fig. 1;
- Fig. 3: eine Darstellung gemäß Fig. 2, jedoch mit abweichend gestaltetem Einspannrand des Siebteils;
- Fig. 4: einen Querschnitt durch das Siebteil alleine;
- Fig. 5: eine Draufsicht auf das Siebteil gemäß Fig. 4 in der Ansicht von oben;
- Fig. 6: eine Darstellung gemäß Fig. 4, jedoch mit abweichend gestaltetem Einspannrand;
- Fig. 7: eine Draufsicht auf den Gegenstand gemäß Fig. 6, gesehen von oben.

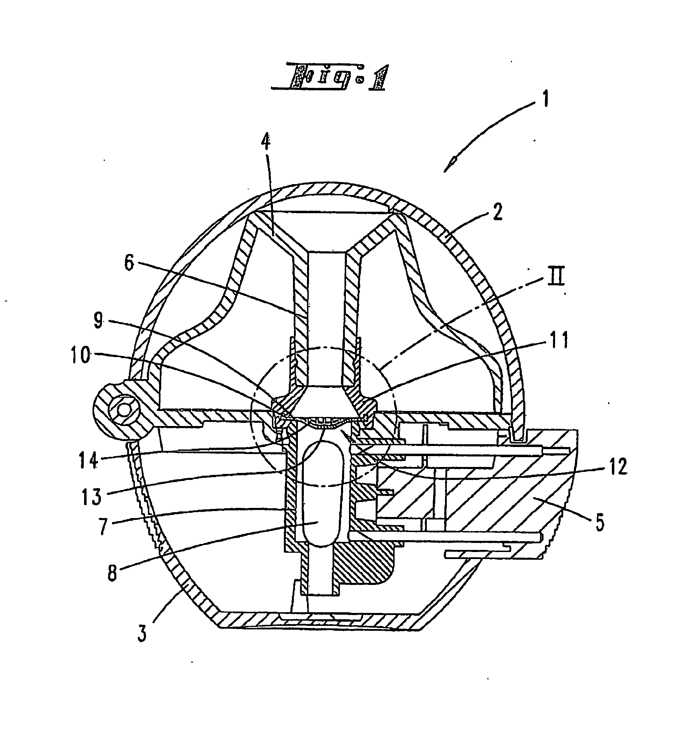

Mit Bezug zu Fig. 1 ist ein Pulverinhalator im Querschnitt dargesteht, wie grundsätzlich etwa aus der bereits eingangs genannten WO 2004/062716 A1 bekannt ist. Zu weiteren Einzelheiten wird auf die genannte Druckschrift verwiesen.

Der Inhalator 1 weist ein Abdeckteil 2 auf, eine Aufnahmegehäuse 3, ein Mundstück 4 und eine Betätigungstaste 5.

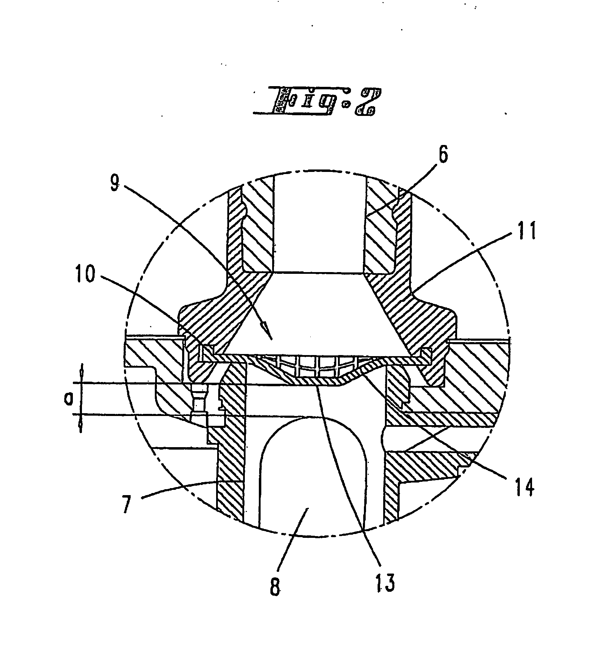

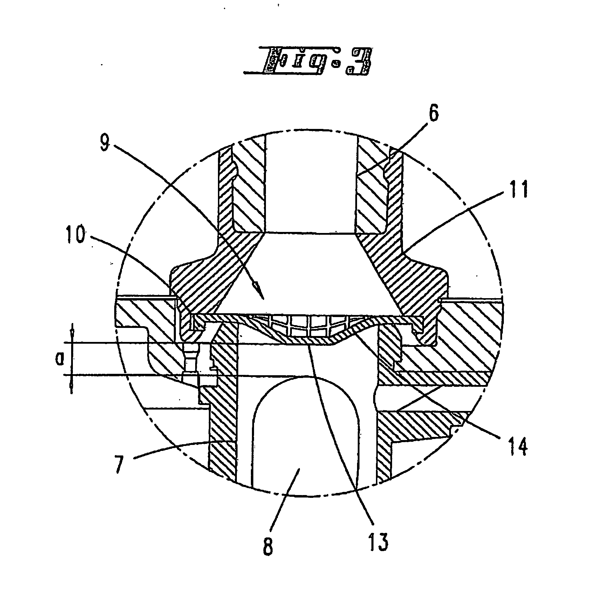

An das Mundstück 4 schließt sich innenseitig ein Saugluftkanal 6 an, der in eine Aufnahmekammer 7 übergeht, in welchem sich ein Substanzvorratsbehältnis 8 befindet. Zwischen der Aufnahmekammer 7 und dem Saugluftkanal 6 ist ein Siebteil 9 angeordnet, das vermittels eines Einspannrandes 10 in einem Adapterteil 11 aufgenommen ist. Ein freier Siebbereich S befindet sich innerhalb des Einspannrandes 10. Das Adapterteil 11 stellt auch einen Teilabschnitt des Saugluftkanals 6. Wie sich insbesondere auch aus den Figuren 2 und 3 ergibt, weist das Siebteil 9 einen Vorstandsbereich 12 auf, der eine Abflachung 13 besitzt.

Der Vorstandsbereich 12 ist um ein Vorstandsmaß v aus der Ebene E, die durch den Einspannrand 10 in den Bereich geht, in dem der Siebbereich S in diesen übergeht, herausgehoben. Das Vorstandsmaß v entspricht 10 bis 20% der Abmessung einer Querschnittsabmessung L (als gerade Querschnittslinie gesehen). Weiter bevorzugt etwa 15%. In die Offenbarung des genannten Bereichs von 10 bis 20% sind auch alle Zwischenwerte, insbesondere in 1/10% - Schritten, im Hinblick auch auf eine Einengung der Bereichsangabe von unten und/oder oben um jeweils 1/10 oder mehr Prozent, eingeschlossen.

Das Siebteil 9 ist insgesamt aus einem Drahtgewebe gebildet, wobei die Querschnittsdarstellungen der Fig.1 bis 3 und 4, 6 jeweils einen Drahtmittig durchschneiden.

Das Siebteil 9 ist weiterhin kreisförmig gestaltet.

Wie sich ebenfalls insbesondere aus den Fig. 2 und 3 ergibt, kann die Abwinklung des Einspannrandes 10 einmal entgegengesetzt zu dem Vorspannbereich 12 und zum anderen auch in Richtung der durch den Vorstandsbereich 12 gegebenen Auswölbung des Siebteils 9 geformt sein. Der Einspannrand 10 kann eine Umspritzung des Drahtgewebes aufweisen, kann aber auch nur durch das Drahtgewebe selbst gebildet sein.

Für die betriebliche Wirksamkeit des Inhalators ist wesentlich, dass der Abstand a, siehe etwa Fig. 3, zwischen einem oberen Ende des unbewegten, in einer Ausgangsstellung befindlichen Substanz-Vomtsbehältnisses 8 und dem insofern nächstliegenden Bereich, hier der Abflachung 13, des Siebtells 9, sehr genau definiert ist und auch in Anbetracht von Herstellungstoleranzen, insbesondere des Siebteils 9 sehr genau eingehalten werden kann. Dies ist durch die Abflachung 13 gegeben. Angestrebt ist eine Maßtoleranz die der Hälfte oder weniger der Drahtdicke des Siebteils 9 entspricht.

Zudem kommt es bei Berührungen zwischen dem Substanz-Vorratsbehältris 8 und dem Siebteil 9 auf Grand der damit gegebenen stabileren Gestaltung auch weniger zu unerwünschten Beeinträchtigungen der Maßhaltigkeit.

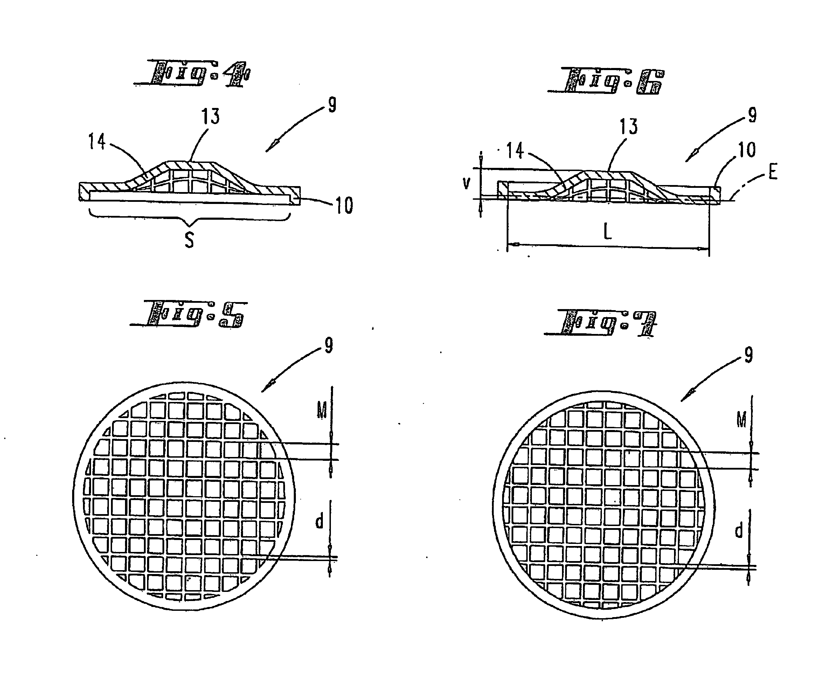

Wie in weiterer Einzelheit auch Fig. 4 und Fig. 6 zu entnehmen ist, schließt sich an die Abflachung 13 nach radial außen ein annähernd gerade verlaufender Übergangsbereich 14, der entsprechend der grundsätzlichen Kreisform des Siebteils 9 umlaufend gegeben ist und damit kegelförmig ist, an.

Die Maschenweite M, wie sie in Bezug auf Fig. 5 oder Fig. 7 dargestellt ist, liegt bei 0,8 mm, während eine Dicke d eines Drahtes, wie er für das Siebgewebe verwendet ist, bei 0,25 mm liegt.

Alle offenbarten Merkmale sind (für sich) erfindungswesentlich.

## Patentansprüche

1. Siebteil (9) für einen Inhalator (1), wobei das Siebteil (9) einen Einspannrand (10) und einen in einem Querschnitt innerhalb des Einspannrandes (10) verlaufenden Siebbereich aufweist, wobei weiter der Siebbereich ausgewölbt ist, so dass ein zu einer Seite hin ausladend geformten Vorstandsbereich (12) entsteht, **dadurch gekennzeichnet, dass** der Vorstandsbereich (12) eine Abflachung (13) aufweist.

2. Siebteil (9) nach Anspruch 1, **dadurch gekennzeichnet, dass** der Vorstandsbereich (12) in der Querschnittsbetrachtung quer zu einer durch den Einspannrand verlaufenden Ebene über den Einspannrand (10) hinausragt.

3. Siebteil (9) nach einem oder mehreren der vorhergehenden Ansprüche, **dadurch gekennzeichnet, dass** die Abflachung (13) in der Querschnittsbetrachtung mittig bezüglich des Siebbereiches ist.

4. Siebteil (9) nach einem oder mehreren der vorhergehenden Ansprüche, **dadurch gekennzeichnet, dass** die Abflachung (13) bezogen auf eine gerade Querschnittslinie 5% oder mehr des innerhalb des Einspannrandes (10) verlaufenden Siebbereiches umfasst.

5. Siebteil (9) nach einem oder mehreren der vorhergehenden Ansprüche, **dadurch gekennzeichnet, dass** der Siebbereich oder das Siebteil (9) insgesamt aus metallischen Drähten gefertigt ist

6. Siebteil (9) nach einem oder mehreren der vorhergehenden Ansprüche, **dadurch gekennzeichnet, dass** der Siebbereich oder das Siebteil (9) insgesamt aus einem Drahtgewebe besteht.

7. Siebteil (9) nach einem oder mehreren der vorhergehenden Ansprüche, **dadurch gekennzeichnet, dass** der Siebbereich eine Maschenweite von 0,4 mm oder mehr aufweist.

8. Siebteil (9) nach einem oder mehreren der vorhergehenden Ansprüche, **dadurch gekennzeichnet, dass** der Siebbereich eine Maschenweite von 1 mm oder weniger aufweist.

9. Siebteil (9) nach einem oder mehreren der vorhergehenden Ansprüche, **dadurch gekennzeichnet, dass** der Siebbereich eine Maschenweite von 0,6 bis 1,0 mm aufweist.

10. Siebteil (9) nach Anspruch 5 oder 6, **dadurch gekennzeichnet, dass** der Draht einen Durchmesser zwischen 0,1 und 0,5 mm aufweist.

11. Inhalator (1) für pulverförmige Substanzen, mit einem zu einem Mundstück (4) führenden Saugluftkanal (6), weiter einem in einer Aufnahmekammer (7) vorgesehenen Substanz-Vorratsbehältnis (8) und einem Siebteil (9) nach einem der Ansprüche 1 bis 10, wobei das Siebteil (9) in dem Saugluftkanal (6) zwischen der Aufnahmekammer (7) und dem Mundstück (4) angeordnet ist und das Siebteil (9) einen Einspannrand (10) aufweist und einen in einem Querschnitt innerhalb des Einspannrandes (10) verlaufenden Siebbereich, wobei der Siebbereich ausgewölbt ist, so dass ein zu einer Seite hin ausladend geformten Vorstandsbereich (12) entsteht und der Vorstandsbereich (12) eine Abflachung (13) aufweist.

## Claims

1. Sieve part (9) for an inhaler (1), said sieve part (9) having a retaining edge (10) and a sieve area extending over a cross-sectional area within the retaining edge (10), the sieve area further being convex, so that a protruding area (12) shaped to project to one side is formed, **characterised in that** the protruding area (12) has a flat portion (13).

2. Sieve part (9) according to claim 1, **characterised in that** the protruding area (12), viewed in cross-section, projects over the retaining edge (10) at right-angles to a plane extending the retaining edge.

3. Sieve part (9) according to one or more of the preceding claims, **characterised in that** the flat portion (13) is central with respect to the sieve area, viewed in cross-section.

4. Sieve part (9) according to one or more of the preceding claims, **characterised in that** the flat portion (14) comprises 5% or more of the sieve area extending within the retaining edge (10), based on a straight cross-sectional line.

5. Sieve part (9) according to one or more of the preceding claims, **characterised in that** the sieve area or the sieve part (9) as a whole is made of metal wires.

6. Sieve part (9) according to one or more of the preceding claims, **characterised in that** the sieve area or the sieve part (9) as a whole consists of a wire mesh.

7. Sieve part (9) according to one or more of the preceding claims, **characterised in that** the sieve area has a mesh size of 0.4 mm or more.

8. Sieve part (9) according to one or more of the preceding claims, **characterised in that** the sieve area has a mesh size of 1 mm or less.

9. Sieve part (9) according to one or more of the preceding claims, **characterised in that** the sieve area has a mesh size of 0.6 to 1.0 mm.

10. Sieve part (9) according to claim 5 or 6, **characterised in that** the wire has a diameter of between 0.1 and 0.5 mm.

11. Inhaler (1) for powdered substances, having a suction air channel (6) leading to a mouthpiece (4), furthermore a substance supply container (8) arranged in a receiving chamber (7), and a sieve part (9) according to one of claims 1 to 10, the sieve part (9) being disposed in the suction air channel (6) between the receiving chamber (7) and the mouthpiece (4) and the sieve part (9) comprising a retaining edge (10) and a sieve area extending in a cross-section within the retaining edge (10), the sieve area being convex, so that a protruding area (12) shaped to project to one side is formed and the protruding area (12) has a flat portion (13).

## Revendications

1. Élément filtrant (9) pour un inhalateur (1), l'élément filtrant (9) présentant un bord de serrage (10) et une zone de filtrage s'étendant dans une section transversale dans le bord de serrage (10), la zone de filtrage étant en outre courbée de sorte qu'une zone débordante (12) formée en saillie vers un côté apparaisse, **caractérisé en ce que** la zone débordante (12) présente un aplatis-sement (13).

2. Élément filtrant (9) selon la revendication 1, **caractérisé en ce que** la zone débordante (12) dépasse du bord de serrage (10), transversalement à un plan s'étendant dans le bord de serrage vu en section transversale.

3. Élément filtrant (9) selon l'une ou plusieurs quelconques des revendications précédentes, **caractérisé en ce que** l'aplatissement (13) est au milieu par rapport à la zone de filtrage, vu en section transversale.

4. Élément filtrant (9) selon l'une ou plusieurs quelconques des revendications précédentes, **caractérisé en ce que** l'aplatissement (13) comporte 5 % ou plus de la zone de filtrage s'étendant dans le bord de serrage (10) par rapport à une ligne de section transversale droite.

5. Élément filtrant (9) selon l'une ou plusieurs quelconques des revendications précédentes, **caractérisé en ce que** la zone de filtrage ou l'élément filtrant (9) est globalement fabriqué en fils métalliques.

6. Élément filtrant (9) selon l'une ou plusieurs quelconques des revendications précédentes, **caractérisé en ce que** la zone de filtrage ou l'élément filtrant (9) se compose globalement d'un tissu du fil métallique.

7. Élément filtrant (9) selon l'une ou plusieurs quelconques des revendications précédentes, **caractérisé en ce que** la zone de filtrage présente une ouverture de mailles de 0,4 mm ou plus.

8. Élément filtrant (9) selon l'une ou plusieurs quelconques des revendications précédentes, **caractérisé en ce que** la zone de filtrage présente une ouverture de mailles d'1 mm ou moins.

9. Élément filtrant (9) selon l'une ou plusieurs quelconques des revendications précédentes, **caractérisé en ce que** la zone de filtrage présente une ouverture de mailles de 0,6 à 1 mm.

10. Élément filtrant (9) selon la revendication 5 ou 6, **caractérisé en ce que** le fil métallique présente un diamètre compris entre 0,1 et 0,5 mm.

11. Inhalateur (1) pour substances poudreuses, avec un canal d'aspiration d'air (6) menant à un embout buccal (4), en outre avec un réservoir de stockage de substance (8) prévu dans une chambre de réception (7) et un élément filtrant (9) selon l'une quelconque des revendications 1 à 10, l'élément filtrant (9) étant disposé dans le canal d'aspiration d'air (6) entre la chambre de réception (7) et l'embout buccal l (4) et l'élément filtrant (9) présentant un bord de serrage (10) et une zone de filtrage s'étendant dans une section transversale dans le bord de serrage (10), la zone de filtrage étant courbée de sorte qu'une zone débordante (12) formée en saillie vers un côté apparaisse et la zone débordante (12) présente un aplatissement (13).
